Europäisches Patentamt

European Patent Office

Office européen des brevets

(11) Publication number: **0 271 713**
**A2**

## EUROPEAN PATENT APPLICATION

(21) Application number: 87116654.2

(22) Date of filing: 11.11.87

(51) Int. Cl.4: **G01N 33/52** , G01N 33/72 ,
C12Q 1/28

(30) Priority: 15.12.86 IL 80964

(43) Date of publication of application:
22.06.88 Bulletin 88/25

(84) Designated Contracting States:
AT BE CH DE ES FR GB IT LI NL SE

(71) Applicant: SAVYON DIAGNOSTICS LTD.
1 Jabotinsky Street
Ramat-Gan(IL)

(72) Inventor: Ben-Michael, Abraham
8, Rehavat Ilan Street Ramat-Ilan
Givat-Shmuel(IL)

(74) Representative: Perani, Aurelio et al
c/o JACOBACCI-CASETTA & PERANI S.N.C.
Via Visconti di Modrone 7
I-20122 Milano(IT)

(54) Method for preparing aqueous solutions containing chromogenic materials.

(57) A method is described by means of which aqueous solutions containing chromogenic materials can be prepared from substantially anhydrous compositions containing all elements of the desired chromogencontaining aqueous solution, water excluded. The invention also encompasses the stable anhydrous compositions for carrying out the method, and aqueous compositions obtained thereby.

The anhydrous compositions, as well as the aqueous compositions, present improved stability characteristics.

# METHOD FOR PREPARING AQUEOUS SOLUTIONS CONTAINING CHROMOGENIC MATERIALS

The present invention relates to a method for preparing aqueous solutions containing chromogenic materials or chromogenic materials and peroxides, to chemical compositions therefor and to aqueous chemical compositions obtained thereby.

In the Israeli patent applications Nos. 74204 and 77299 of the same applicant there are described stable chemical compositions, particularly suitable for diagnostic purposes, which contain chromogen materials or both chromogen materials and a peroxide. The said compositions are surprisingly stable since chromogenic materials are usually unstable even in the absence of a peroxide, and mixtures of chromogenic materials and peroxides are known in the art to be particularly unstable and to react to give a color reaction, which may be catalyzed by various agents such as UV light and metal ions. The said stable solutions comprise a water miscible organic solvent, an aqueous solution and a stabilizing compound, to which the chromogen or the chromogen/peroxide mixture is added. Among the stabilizing compounds described in the abovementioned patent applications there are the so-called TuOcm compounds.

The art is constantly searching for ways to improve the stability of chromogen/peroxides mixtures, in order to simplify and reduce costs of diagnostic routines and to allow to prepare large amounts of ready-to-use working solutions. A considerable drawback of the solutions employed in the art lies in that a large amount of the final working solution - 50% or more - consists of water, which obviously, increases shipping and storage costs. Another problem related to the above solutions is that particularly unstable chromogens, such as benzidine, 3-amino-9-ethylcarbazole and 3,3'-diaminobenzidine derivatives, are very difficult to stabilize, and chromogenic systems such a p-amino-p methoxy diphenylamine/ 4-aminoantipyrine cannot be effectively stabilized even in the exceptionally stable solutions of the above mentioned patent applications Nos. 74204 and 77299, and they spontaneously react with hydrogen peroxide at room temperature, within 3 to 5 hours.

Throughout this specification, a chromogen material is defined as a material which changes its color when in the presence of a peroxide of the formula ROOR', which is decomposed by a peroxide decomposing agent. R and R' are defined as being each independently hydrogen or an organic substitutent which can suitably form an organic peroxide or hydroperoxide. Examples of peroxides are hydrogen peroxide, 2-butanone peroxide and cumene hydroperoxide. Examples of peroxide decomposing agents are UV light, metal ions, active enzymes like peroxidase or catalase and pseudo-peroxidases like haemoglobin or Cytochrom-C.

It has now been most surprisingly found, and this is an object of the present invention, that it is possible to provide substantially anhydrous concentrated chromogen solutions, even in admixture with a peroxide, which are stable for surprisingly long periods of time.

It has also been surprisingly found, and this is a further object of the invention, that it is possible to dissolve buffering components and chelating agents in a substantially anhydrous water-miscible organic solvent, which components and agents were not considered to be soluble in a substantially anhydrous environment.

It has further been surprisingly found, and this is another object of the invention, that when an aqueous medium is added to the concentrated solutions of the invention, a working solution is obtained whose activity is comparable to that of a freshly-prepared working solution.

It is an object of the invention to provide substantially anhydrous solutions containing all the desired components of a chromogen/peroxide working solution, water excluded, to which water can be added in a predetermined proportion to obtain ready-to-use chromogen or chromogen/peroxide containing working solutions.

As it will be apparent to a person skilled in the art, it is quite surprising that a chromogen material and a peroxide that, when brought into contact in working solutions react very quickly, causing the working solution to degenerate very rapidly, may coexist for very long periods of time in the compositions of the invention, without any appreciable deterioration. Even more surprising, as it will be apparent hereafter, is that this result may be achieved without the need for any stabilizing compound and, still more surprising, that this can be achieved when the peroxide employed is $H_2O_2$, which is known to be extremely reactive and to decompose very quickly, thereby priming the chromogenic reaction.

It is another object of the invention to provide a method, employing the aforementioned concentrated solutions, by which mixtures of chromogenic materials and peroxides can be stabilized for long periods of time.

The method for preparing aqueous solutions containing chromogenic materials is characterized by the steps of:

(a) providing a substantially anhydrous liquid composition comprising, in a water-miscible organic solvent, a chromogenic material or a mixture of two or more chromogenic material(s) and an effective amount of chelating agents, as hereinafter defined, alone or in admixture with one or more peroxide(s) and/or buffering components and/or stabilizing compounds; and

   (b) reconstituting the chromogen-containing solution by adding water to the said composition (a).

By substantially anhydrous it is meant that no appreciable amounts of water are present in the composition of the invention. However, as it will be apparent to a person skilled in the art, it is possible that trace amounts of water will be present in the composition, because of imperfect drying of the components, or even as water taken up from atmospheric air during handling. Small amounts of water do not appreciably affect the compositions of the invention. In fact, the inventors have found that the absence of water substantially "freezes" the shelf life of the concentrated composition, and that its absence permits to obtain activities of the reconstituted working solution - after water has been added - that are comparable to the activity of a freshly-prepared working solution.

By an effective amount of chelating agents it is meant an amount below which the stabilizing effect of chelation is not achieved. Typically, a minimal amount of 100 mg of chelating agents is required for each 1000 ml of anhydrous solution. Preferred chelating agents content are those which comprise, for each 1000 ml of anhydrous solution, at least about 100 mg of each of acetanilide, sodium pyrophosphate and 8-hydroxyquinoline hemi-sulfate salt, and about 1000 mg of citrate.

The substantially anhydrous liquid stable composition for carrying out the method of the invention is characterized in that it contains, in a water-miscible organic solvent, a chromogenic material or a mixture of two or more chromogenic material(s) and an effective amount of chelating agents, as defined in the specification, alone or in admixture with one or more peroxide(s) and/or buffering components and/or stabilizing compounds, the said composition being stable in the anhydrous state for at least 6 months at 25°C and being capable of stabilizing a mixture of a chromogenic material and a peroxide for at least 2 months at 25°C, when reconstituted to the form of an aqueous working solution by the addition of water.

According to a preferred embodiment of the invention, the said anhydrous composition further comprises one or more peroxides(s).

According to a preferred embodiment of the invention the peroxide is hydrogen peroxide. According to another preferred embodiment of the invention the peroxide is cumene hydroperoxide.

The composition of the invention may further comprise one or more stabilizing compound(s).

While such stabilizing compounds are not necessary for obtaining stable compositions of the invention, they are useful for providing improved shelf-lives of the reconstituted working solutions.

According to a preferred embodiment of the invention the said anhydrous composition further comprises buffering components.

The water-miscible organic solvent is preferably selected from among methanol, ethanol, dioxane, dimethylsulfoxide, sulfolane, acetamide, hexamethylphosphoric triamide, N-methylacetamide, N,N'-dimethylacetamide, dimethylformamide and formamide.

Any stabilizing compound may be employed, whenever desired, such as the TuOcm compounds and heterocyclic compounds. The TuOcm compounds, described in Israeli Patent Application No. 74204, are defined as being compounds comprising at least two unsaturations and at least one cyclic moiety, which unsaturations may be either ethylenic double bonds or aromatic or heteroaromatic unsaturations. However, other stabilizing compounds, having beneficial effect on the stability of the aqueous working solution, can be usefully employed together with the compositions of the invention.

Examples of preferred TuOcm compound are 8-hydroxyquinoline, 4-chloro-7-(trifluoromethyl) quinoline, 5-chloro-8-hydroxyquinoline, the hemi-sulfate salt of 8-hydroxyquinoline, guaiacol sulfonic acid, vanillin, vanillic acid, N-benzylidene methylamine, N-benzylidenebenzylamine· 10-benzylidene-9-anthrone, 2-(2-hydroxyphenyl)-benzoxazole, 1-hydroxybenzotriazole, 2-hydroxy-benzophenone and 2,4,4'-trihydroxy-benzophenone, flavianic acid, o-toluamide, p-toluamide, m-toluamide, p-nitrobenzamide, salicylanilide, 2,4,6-collidine, 2-quinoxalinol, 4-methylpyrimidine, benzamide, 4'-nitroacetanilide, ethyl nicotinate, 2-amino-4-6-dimethylpyrimidine, 2-(aminomethyl)-pyridine, 4-pyridine carboxylic acid, 2,6-lutin, 2-amino-6-methylpyridine, or a mixture of two or more of such stabilizing compounds;

Examples of preferred heterocylic compounds are: 2-pyrrolidinone, 1-methyl-2-pyrrolidinone, piperidine, methyl-piperidine, 4-piperidnopiperidine, 4-ethylpiperidinopiperidine, morpholine, propylmorpholine, 1-ethylpiperidine, 1-(2-aminoethylpiperazine), 12-crown-4, 2,5-diethoxytetrahydrofuran, 1,4-dimethylpiperazine, dithiane, 4-ethylmorpholine and oxepane.

Preferred buffering components to be used together with the compositions of the invention are: phosphate, acetate, acetate/citrate, glycine, borate, tris(hydroxymethyl)aminomethane (TRIS), N, N-bis-[2-hydroxyethyl]-2-aminoethane-sulfonic acid (BES), N,N-bis[2-hydroxyethyl]-glycine (BICINE), N-2-hydroxyethylpiperazine-N'-2-ethanesulfonic

acid (HEPES), 2-[N-morpholino]ethanesulfonic acid (MES), 3-[N-morpholino] ethanesulfonic acid (MOPS), N-tris[hydroxymethyl]methyl-2-aminoethanesulfonic acid (TES) and N-tris-[hydroxymethyl] methylglycine (TRICINE).

Preferred chromogen materials comprise 4-chloro-1-naphthol, 2,4-dichloro-1-naphthol, benzidine•2HCl, 3,3'-diaminobenzidine•4HCl, 3,3',5,5'-tetramethylbenzidine, 3,3',5,5'-tetramethylbenzidine•2HCl, 3,3',5,5'-tetramethylbenzidine•2HCl•2H$_2$O, 3-amino-9-ethylcarbazole, o-tolidine, o-dianisidine, guaiacol, 4-aminoantipyrine/ alpha-naphthol, 2,2'-azino-bis-(3-ethylbenzothiazoline-6-sulfonic acid), 3-methyl-2-benzothizolinone hydrazone hydrochloride, 3,5-diaminobenzoic acid, 3,4-diaminobenzoic acid, phenothiazine, 4-dimethylaminobenzoic acid, 1-bromo-2-naphtol, 8-amino-2-naphthol, 1-amino-4-chloronaphthol, diphenylamine, 4-nitro-1,2-phenylenediamine, 5-aminosalyscylic acid, pyrogallol, 8-amino-1-haphthol-5-sulfonic acid, p-aminodiphenylamine, p-amino-p-methoxy diphenylamine, 1,2-phenylenediamine, p-phenylenediamine, o-phenylenediamine, o-phenylenediamine•HCl, N,N,N',N'-tetramethyl-p-phenylenediamine•2HCl, syringaldazine and m-phenylenediamine.

The method for preparing a chromogen/peroxide containing working solution according to the invention is characterized by the steps of:

a) providing a substantially anhydrous liquid composition comprising, in a water-miscible organic solvent, a chromogenic material or a mixture of two or more chromogenic material(s) and an effective amount of chelating agents, as hereinbefore defined;

b) adding a peroxide to the said composition (a); and

c) adding water to obtain the desired predetermined dilution of the chromogen in the working solution;

steps (b) and (c) being performed in any appropriate order.

The final working wolution, after reconstitution has been effected by the addition of water, may contain between 10% and 80% by volume of the concentrated solution, preferably 20% to 50%.

Working solutions containing a chromogen material and a peroxide, in a mixture .of a water-miscible organic solvent and water, whenever obtained by the compositions or method of the invention, also form part of the present invention.

It is clear that the concentrated solutioins of the invention provide a very convenient way to prepare, ship and store different kinds of working solutions, which can be tailored to the specific requirements of the user. For instance, if the final working solution is not to be kept for long periods of time after its reconstitution, the stabilizing compound can be dispensed with. Thus, it is possible to desk-store the concentrated solution of the invention and to withdraw any amount thereof at any time, and use it instantly by adding to it the appropriate amount of water.

The above and other characteristics and advantages of the invention will be better understood through the following illustrative and non-limitative examples thereof. Testing of the solution is effected by reaction with horseradish peroxidase, as the test peroxidase. Solutions not containing H$_2$O$_2$ or 2-butanone peroxide were checked by adding first H$_2$O$_2$ and immediately thereafter the horseradish peroxidase. Compositions containing Cumene hydroperoxide were tested by using haemoglobin, as the test pseudo-peroxidase.

Stability of an anhydrous composition is evaluated by reconstituting it by the addition of water and comparing its activity with the appropriate test peroxidase, as hereinbefore detailed, to the activity of an identical but freshly-prepared composition, by colorimetric evaluation. Likewise, stability of an aqueous working solution at any given time after reconstruction, is checked by comparison with an identical - but freshly prepared - composition.

Example 1

2000 mg of 4-chloro-1-naphthol were dissolved into 200 ml of dimethylsulfoxide. The resulting mixture was stirred for 2 hours. At the end of this period, 500 microliters of H$_2$O$_2$ were added, and the mixture was stirred for additional 2 hours.

The resulting solution was stable for at least 3 months at 37°C, 9 months at 25°C and 24 months at 4°C. Stability was checked by reconstituting the working solution by the addition of 4 volumes of distilled water per volume of concentrated solution, and by checking its activity as compared to the activity of a freshly prepared identical solution.

Example 2

2000 mg of 4-chloro-1-naphthol, 320 mg of acetanilide, 320 mg of pyrophosphate and 3200 mg of citrate were dissolved into 200 ml of dimethylsulfoxide. Care was taken to allow complete dissolution of each component before the next component was added. The resulting mixture was stirred for 2 hours. At the end of this period, 500 microliters of H$_2$O$_2$ were added, and the mixture was stirred for additional 2 hours.

The resulting solution was stable for a least 3 months at 37°C, 9 months at 25°C and 24 months at 4°C.

## Example 3

A composition was prepared as in Example 2, but adding also 3872 mg of phosphate buffering components, prepared using a 1:0.82 w/w mixture of potassium phosphate dibasic ($K_2HPO_4$) and sodium phosphate dibasic ($Na_2HPO_4$).

The resulting solution was stable for 3 months at 37°C, 9 months at 25°C and 24 months at 4°C.

## Example 4

A composition was prepared as in Example 3, but with the further addition 4000 mg of 8-hydroxyquinoline hemi-sulfate salt.

The resulting solution was stable for 3 months at 37°C, 9 months at 25°C and 24 months at 4°C.

## Example 5 (Comparative)

The solution of Example 1 was reconstituted by adding to it 800 ml of distilled water. The resulting working solution was checked for stability and was found to be stable for 7 days at 37°C, 1 month at 25°C and 3 months at 4°C.

## Example 6

The solution of Example 2 was reconstituted by adding to it 800 ml of distilled water. The resulting working solution was checked for stability and was found to be stable for 1 month at 37°C, 3 months at 25°C and 12 months at 4°C.

## Example 7

The solution of Example 3 was reconstituted by adding to it 800 ml of distilled water. The resulting working wolution was checked for stability and was found to be stable for 1 month at 37°C, 3 months at 25°C and 12 months at 4°C.

## Example 8

The solution of Example 4 was reconstituted by adding to it 800 ml of distilled water. The resulting working wolution was checked for stability and was found to be stable for 2 month at 37°C, 6 months at 25°C and 12 months at 4°C.

## Example 9

Examples 1 through 8 were repeated, but using dimethylformamide as the solvent. Comparable - but slightly less good - results were obtained in each case (about 20% less shelf lives).

## Example 10

Examples 1 through 8 were repeated, but using N-methylacetamide as the solvent. Comparable results were obtained in each case.

## Example 11

80 mg of o-dianisidine were dissolved into 20 ml sulfolane. The resulting mixture was stirred for 2 hours. At the end of this period, 10 microliters of $H_2O_2$ were added and the mixture was stirred for additional 2 hours. The resulting solution was stable for 1 month at 37°C, 3 months at 25°C and 12 months at 4°C.

## Example 12

A solution as in Example 11 was prepared, which further contained 200 mg of citric acid trisodium salt, 20 mg of acetanilide, 20 mg of sodium pyrophosphate decahydrate, 40 mg of 8 hydroxyquinoline and 400 mg of 2,3-dinitro-1-naphthol-7 sulfonic acid. The resulting solution was stable for 3 months at 37°C, 6 months at 25°C and 12 months at 4°C.

## Example 13

The solution of Example 12 was reconstituted by adding to it 80 ml of distilled water. The resulting solution was stable for 1 month at 37°C, 4 months at 25°C and 9 months at 4°C.

## Example 14

A solution containing 200 mg of 4-aminoantipyrine and 2000 mg of alpha-naphthol, together with 200 mg of citric acid tri-sodium salt, 20 mg of acetanilide and 20 mg of sodium pyrophosphate decahydrate were dissolved in 100 ml of N,N-dimethylacetamide. The resulting mixture was stirred for 2 hours. The resulting solution was stable for 3 months at 37°C, 6 months at 25°C and 12 months at 4°C.

## Example 15

The solution of Example 14 was reconstituted by adding to it 300 ml of distilled water. The resulting solution was stable for 1 month at 37°C, 5 months at 25°C and 10 months at 4°C.

## Example 16

Examples 14 and 15 were repeated, using 2000 mg of 3,3',5,5'-tetramethylbenzydine instead of 4-aminoantipyrine and alpha-naphthol. The results obtained were as in Examples 14 and 15.

## Example 17

Operating as in Example 1, but employing 200 mg of guaiacol in 160 ml of dimethylsulfoxide, and using 40 microliters of $H_2O_2$, a composition was obtained which was stable for 3 months at 37°C, 9 months at 25°C and 18 months at 4°C.

## Example 18

The composition described in Example 17 was prepared, with the addition of 16 mg of acetanilide, 16 mg of sodium pyrophosphate, 40 mg of 8-hydroxyquinoline and 400 mg of 8-hydroxyquinoline hemi-sulfate salt. The resulting solution was stable for 3 months at 37°C, 12 months at 25°C and 24 months at 4°C.

## Example 19

The composition of Example 18 was reconstituted, by adding to it 240 ml of tap water. The resulting solution was stable for 1 month at 37°C, 3 months at 25°C and 9 months at 4°C.

## Example 20

A composition was prepared, according to the procedure of the preceding examples, which contained 400 mg aminoethylcarbazole, 400 mg 2-amino-4-picoline, 400 mg sodium pyrophosphate, 400 mg sodium citrate and 400 mg of acetanilide in 200 ml DMSO. 120 microliters of $H_2O_2$ were employed. The resulting solution was stable for 3 months at 37°C, 9 months at 25°C and 18 months at 4°C.

The solution was reconstituted by adding to it 800 ml of distilled water., The resulting solution was stable for 1 month at 37°C, 4 months at 25°C and 9 months at 4°C.

## Example 21

Example 20 was repeated, but using 400 microliters of 2-butanone peroxide, instead of $H_2O_2$. Stabilities obtained as in Example 20.

## Example 22

Example 20 was repeated, but using 400 microliters of Cumene hydroperoxide, instead of $H_2O_2$. Stabilities obtained as in Example 20.

The above description and examples have been provided for the purpose of illustration, and are not intended to be limitative. Many variations can be effected in the various compositions, methods and procedures, without exceeding the scope of the invention.

**Claims**

1. A method for preparing aqueous solutions containing chromogenic materials, characterized by the steps of:

a) providing a substantially anhydrous liquid composition comprising, in a water-miscible organic solvent, a chromogenic material or a mixture of two or more chromogenic material(s) and an effective amount of chelating agents as defined in the specification, alone or in admixture with one or more peroxide(s) and/or buffering components and/or stabilizing compounds; and

b) reconstituting the chromogen-containing solution by adding water to the said composition (a).

2. A method according to claim 1, characterized in that the composition (a) further comprises one or more peroxide(s).

3. A method according to claim 2, characterized in that the peroxide is hydrogen peroxide.

4. A method according to claim 2, characterized in that the peroxide is cumene hydroperoxide.

5. The method of claim 2, characterrized in that the composition (a) further comprises one or more stabilizing compound(s).

6. The method of claim 2 or 5, characterized in that the composition (a) further comprises buffering components.

7. The method of any one of claims 1 to 6, characterized in that the water-miscible organic solvent is selected from among methanol, ethanol, dioxane, dimethylsulfoxide, sulfolane, acetamide, hexamethylphosphoric triamide, N-mthylacetamide, N,N'-dimethylacetamide, dimethylformamide and formamide.

8. The method of claim 5, characterized in that the stabilizing compound is selected from among a TuOcm compound, as defined in the specification, and a heterocyclic compound.

9. The method of claim 8, wherein the TuOcm compound is selected from among 8-hydroxyquinoline, 5-chloro-8-hydroxyquinoline, the hemisulfate salt of 8-hydroxyquinoline, guaiacol sulfonic acid, vanillin, vanillic acid, N-benzylidene methylamine, N-benzylidenebenzylamine 10-benzylidene-9-anthrone, 2-(2-hydroxyphenyl)-benzoxazole, 1-hydroxybenzotriazole, 2-hydroxy-benzophenone and 2,4,4'-trihydroxy-benzophenone, flavianic acid, o-toluamide, p-toluamide, m-toluamide, p-nitrobenzamide, salicylanilide, 2,4,6-collidine, 2-quinoxalinol, 4-methylpyrimidine, benzamide, 4'-nitroacetanilide, ethyl nicotinate, 2-amino-4-6-dimethylpyrimidine, 2-(aminomethyl)-pyridine, 4-pyridine carboxylic acid, 2,6-lutidin, 2-amino-6-methylpyridine, or a mixture of two or more of such stabilizing compounds;

10. The method of claim 8, wherein the heterocyclic compounds is selected from among 2-pyrrolidinone, 1-methyl-2-pyrrolidinone, piperidine, methyl-piperidine, 4-piperidinopiperidine, 4-ethylpiperidinopiperidine, morpholine,propylmorpholine, 1-ethylpiperidine, 1-(2-aminoethylpiperazine), 12-crown-4, 2,5-diethoxytetrahydrofuran, 1,4-dimethylpiperazine, dithiane, 4-ethylmorpholine and oxepane.

11. The method of claim 6, characterized in that the buffering component is selected from among phosphate, acetate, acetate/citrate, glycine, borate, tris(hydroxymethyl)aminomethane (TRIS), N, N-bis[2-hydroxyethyl]-2-aminoethane-sulfonic acid (BES), N,N-bis[2-hydroxyethyl]-glycine (BICINE), N-2-hydroxyethylpiperazine-N'-2-ethanesulfonic acid (HEPES), 2-[N-morpholino]-ethanesulfonic acid (MES), 3-[N-morpholino]ethanesulfonic acid (MOPS), N-tris[hydroxymethyl]-methyl-2-aminoethanesulfonic acid (TES) and N-tris[hydroxymethyl] methylglycine (TRICINE).

12. The method of any of one the preceding claims, wherein the chromogen material is selected from among 4-chloro-1-naphthol, 2,4-dichloro-1-naphthol, benzidine•2HCl, 3,3'-diaminobenzidine•4HCl, 3,3',5,5'-tetramethylbenzidine, 3,3',5,5'-tetramethylbenzidine•2HCl, 3,3',5,5'-tetramethylbenzidine•2HCl•2H$_2$O, 3-amino-9-ethylcarbazole, p-hydroxyphenylacetic acid, o-tolidine, o-dianisidine, guaiacol, 4-aminoantipyrine/ alpha-naphthol,2,2'-azino-bis-(3-ethylbenzothiazoline-6-sulfonic acid), 3-methyl-2-benzothiazolinone hydrazone hydrochloride, 3,5-diaminobenzoic acid,3,4-diaminobenzoic acid, phenothiazine, 4-dimethylaminobenzoic acid,1-bromo-2-naphthol, 8-amino-2-naphthol, 1-amino-4-chloronaphthol,diphenylamine, 4-nitro-1,2-phenylenediamine, 5-aminosalyscylic acid, pyrogallol, 8-amino-1-haphthol-5-sulfonic acid, p-aminodiphenylamine,p-amino-p-methoxy diphenylamine, 1,2-phenylenediamine, p-phenylenediamine, o-phenylenediamine, o-phenylenediamine•HCl,N,N,N',N'-tetramethyl-p-phenylenediamine•2HCl, syringaldazine and m-phenylenediamine.

13. The method of any one of the preceding claims, wherein the chelating agents are selected from sodium pyrophosphate, sodium citrate, 8-hydroxyquinoline, 8-hydroxyquinoline hemi-sulfate salt, acetanilide.

14. The method according to any one of the preceding claims, wherein the final working solution contains between 10% and 80% by volume of the concentrated solution, preferably 20% to 50%.

15. A method for preparing a chromogen/peroxide containing working solution characterized by the steps of:

(a) providing a substantially anhydrous liquid composition comprising, in a water-miscible organic solvent, a chromogenic material or a mixture of two or more chromogenic material(s) and an effective amount of chelating agents, as defined in the specification;

b) adding a peroxide to the said composition (a); and

c) adding water to obtain the desired predetermined dilution of the chromogen in the working solution;

steps (b) and (c) being performed in any appropriate order.

16. Working solutions containing a chromogenic material or a chromogenic material and a peroxide, whenever prepared by the method of claim 14 or 15.

17. A substantially anhydrous liquid stable composition for carrying out the method of claim 1, characterized in that it contains, in a water-miscible organic solvent, a chromogenic material or a mixture of two or more chromogenic material(s) and an effective amount of chelating agents, as defined in the specification, alone or in admixture with one or more peroxide(s) and/or buffering components and/or stabilizing compounds, the said composition being stable in the anhydrous state for at least 6 months at 25°C and being capable of stabilizing a mixture of a chromogenic material and a peroxide for at least 2 months at 25°C, when reconstituted to the form of an aqueous working solution by the addition of water.

18. A composition according to claim 17, wherein the peroxide is the peroxide of claim 3 or 4, the water-miscible organic solvents are those defined in claim 7, the stabilizing compounds are those defined in claims 8 to 10, the buffering .components are as defined in claim 11, the chromogenic materials are those defined in claim 12 and the chelating agents are as defined in claim 13.

19. Substantially anhydrous liquid stable compositions comprising, in a water-miscible organic solvent, a mixture of one or more chromogenic materials(s) together with an effective amount of chelating agents, as defined in the specification, essentially as described and illustrated.

20. Substantially anhydrous liquid stable compositions comprising, in a water-miscible organic solvent, a mixture of one or more chromogenic materials(s) together with an effective amount of chelating agents, as defined in the specification, and a peroxide, essentially as described and illustrated.

21. A method for preparing a chromogen/peroxide containing working solution, substantially as described and illustrated.

22. A method for preparing aqueous solutions containing chromogenic materials, essentially as described and illustrated.